# EUROPÄISCHE PATENTSCHRIFT

(11) **EP 0 941 212 B1**
(45) Veröffentlichungstag und Bekanntmachung des Hinweises auf die Patenterteilung: **21.03.2001**
(21) Anmeldenummer: 97952786.8
(22) Anmeldetag: 20.11.1997
(51) Int. Cl.: C07C 29/143, C07C 33/20

(54) **VERFAHREN ZUR KATALYTISCHEN, ENANTIOSELEKTIVEN REDUKTION VON KETONEN**
ENANTIOSELECTIVE CATALYTIC REDUCTION OF KETONES
REDUCTION CATALYTIQUE ENANTIOSELECTIVE DE KETONES

(30) Priorität: 20.11.1996 DE 19647892
(43) Veröffentlichungstag der Anmeldung: 15.09.1999
(73) Patentinhaber: Degussa-Hüls Aktiengesellschaft, 60287 Frankfurt am Main (DE); FORSCHUNGSZENTRUM JÜLICH GMBH, 52425 Jülich (DE)
(72) Erfinder: GIFFELS, Guido, D-53757 St. Augustin-Menden (DE); FELDER, Marcel, D-5605 Dottikon (DE); KRAGL, Udo, D-52428 Jülich (DE); WANDREY, Christian, D-52428 Jülich (DE); DRAUZ, Karlheinz, D-63579 Freigericht (DE); BOMMARIUS, Andreas, D-60596 Frankfurt (DE); BOLM, Carsten, D-52072 Aachen (DE); DERRIEN, Nadine, Liverpool L15 9HL (GB)
(86) Internationale Anmeldenummer: EP9706479
(87) Internationale Veröffentlichungsnummer: WO9822415

(56) Entgegenhaltungen:
- FRANOT C ET AL: "A Polymer-Bound Oxazaborolidine Catalyst: Enantioselective Borane Reductions of Ketones" TETRAHEDRON: ASYMMETRY, Bd. 11, Nr. 6, November 1995, Seite 2755-2766 XP004047982 in der Anmeldung erwähnt
- FELDER M ET AL: "A polymer-enlarged homogeneously soluble oxazaborolidine catalyst for the asymmetric reduction of ketones by borane" TETRAHEDRON: ASYMMETRY, Bd. 12, Nr. 8, 27.Juni 1997, Seite 1975-1977 XP004081466

## Beschreibung

Die Erfindung betrifft ein Verfahren zur katalytischen, enantioselektiven Reduktion von Ketonen zu chiralen Alkoholen.

Chirale Alkohole sind beispielsweise wichtige Intermediate in der pharmazeutischen Industrie. Es besteht daher ein großes Interesse an Verfahren, die diese Verbindungen in hoher optischer Reinheit zur Verfügung stellen. Dabei sind insbesondere katalytische Verfahren vorteilhaft, da mit einer geringen Menge des in der Regel kostspieligen chiralen Auxiliars ein Vielfaches an chiralem Produkt produziert werden kann.

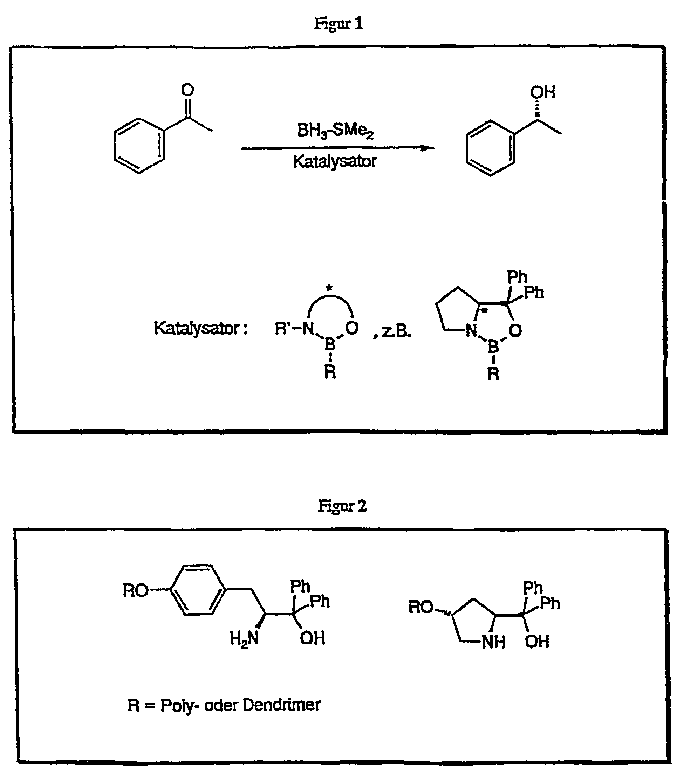

Ein Verfahren dieser Art ist beispielsweise die Oxazaborolidin-katalysierte Reduktion von Ketonen zu chiralen Alkoholen mit Boranen, wie z. B. Boran-Dimethylsulfid-Komplex oder Boran-Tetrahydrofuran-Komplex (vgl. z. B. Wallbaum, S. und Martens, J., in: *Tetrahedron Asymmetrie* 3, 1992, 1475-1508). Die Reaktion ist in Figur 1 wiedergegeben. Diese Methode liefert die chiralen Alkohole in guten bis sehr guten Ausbeuten und Enantiomerenüberschüssen (= enantiomeric excess = ee). Auf diese Weise konnte eine Reihe pharmazeutisch relevanter Verbindungen hergestellt werden.

Üblicherweise wird jedoch zur Erzielung eines optimalen Enantiomerenüberschusses etwa 5 bis 10 mol-% des Katalysators (bezogen auf Keton) benötigt. Eine Minimierung der Katalysatorkosten kann deshalb einen entscheidenden Beitrag zur Wirtschaftlichkeit des Verfahrens leisten.

Es wurden daher bereits vielfache Versuche unternommen, die Zyklenzahl (mol Produkt pro mol verbrauchtem Katalysator) zu erhöhen. So wurden beispielsweise die als Katalysatoren eingesetzten Oxazaborolidine an unlöslichen Trägern immobilisiert. Diese heterogenen Oxazaborolidine wurden durch Ankopplung des verwendeten chiralen Aminoalkohol-Liganden an ein quervernetztes Polystyrolharz mit Boronsäuregruppen erhalten (Franot et al., in: *Tetrahedron Asymmetry* 6*,* 1995, 2755-2766). Der so erhaltene heterogenisierte Katalysator kann nach der Reaktion abfiltriert und erneut eingesetzt werden. Bereits bei der Durchführung des dritten Reaktionszyklus sinkt der erreichte Enantiomerenüberschuß unter 80 % ab, so daß ein weiterer Einsatz des Katalysators nicht mehr sinnvoll ist. Daher konnte auf diese Weise die Zyklenzahl nur geringfügig von 10 (entsprechend 10 mol-% Katalysator) auf 20 bis 30 erhöht werden.

Der vorliegenden Erfindung lag daher das technische Problem zugrunde, ein Verfahren bereitzustellen, das eine effektive Ausnutzung des chiralen Katalysators ermöglicht.

Dieses Problem wird erfindungsgemäß dadurch gelöst, daß die katalytische, enantioselektive Reduktion von Ketonen zu chiralen Alkoholen mit einem molekulargewichtsvergrößerten Katalysator in einem Membranreaktor durchgeführt wird. Mit diesem erfindungsgemäßen Verfahren gelingt es überraschenderweise, die Zyklenzahlen um den Faktor 12 auf ≥ 120 zu steigern, und zwar ohne Verlust der Enantioselektivität des eingesetzten Katalysators. Zudem liefert dieses Verfahren die chiralen Alkohole in Enantiomerenüberschüssen von ≥ 90 % ee. Die Rückhaltung bzw. Abtrennung des löslichen Katalysators durch eine Membran, wie beispielsweise eine Ultra- oder Nanofiltrationsmembran, hat zudem den Vorteil, daß die Reaktion in homogener Lösung ohne Stofftransportlimitierung abläuft.

Als Katalysator wird insbesondere ein chirales Oxazaborolidin eingesetzt. Als Katalysatoren sind aber auch Übergangsmetallverbindungen, wie z. B. Titanate, einsetzbar, die dann über die chiralen Liganden, beispielsweise Diol-Liganden, an die zur Molekulargewichtsvergrößerung verwendete Verbindung angekoppelt werden können.

Das erfindungsgemäß bevorzugte Oxazaborolidin weist 2 mögliche Stellen für eine Molekulargewichtsvergrößerung auf: Es kann eine Ankopplung dieser Substanz an die zur Molekulargewichtsvergrößerung verwendete Verbindung über einen Aminoalkohol oder eine Boronsäure erfolgen. Erfindungsgemäß ist das Oxazaborolidin vorzugsweise über den chiralen Aminoalkohol an die zur Molekulargewichtsvergrößerung verwendete Verbindung angekoppelt.

Als Liganden kommen prinzipiell alle chiralen Aminoalkohole in Frage, die eine (weitere) funktionelle Gruppe besitzen, die eine Anbindung ermöglicht, insbesondere aber Tyrosinol-oder Hydroxyprolinderivate. Speziell Diphenyltyrosinol und Diphenylhydroxyprolinol (Figur 2) erzielen sehr gute Ergebnisse.

Zur Molekulargewichtsvergrößerung wird vorzugsweise ein Polymer, insbesondere ein Polystyrol oder Polysiloxan, eingesetzt, eine Molekulargewichtsvergrößerung der Katalysatoren bzw. ihrer Vorstufen kann aber auch durch die Ankopplung der entsprechenden Verbindungen beispielsweise an Dendrimere erreicht werden. Die Ankopplung erfolgt, indem ein Ligand über eine zur Katalyse nicht benötigte funktionelle Gruppe an ein fertiges Poly- oder Dendrimer gebunden wird. Alternativ kann der Ligand auch mit einer polymerisierbaren Funktionalität versehen und mit einem anderen Monomer copolymerisiert werden.

Die an der erfindungsgemäßen katalytischen, enantioselektiven Reduktion von Ketonen beteiligten Substanzen sollten möglichst in organischen Lösungsmitteln homogen löslich sein.

Aus den chiralen Aminoalkoholen bildet sich in Gegenwart des (zur Reduktion verwendeten) Borans ("BH₃") unter Abspaltung von zwei Äquivalenten Wasserstoff der aktive Oxazaborolidinkatalysator. Dieses kann in *situ* im Reaktor erfolgen oder vor dem Einspülen des Katalysators in den Reaktor separat erfolgen.

Auch die Reaktion der Aminoalkohol-Liganden mit verschiedenen Boronsäuren oder Boronsäurederivaten kann zur Darstellung von Oxazaborolidinen herangezogen werden. Die molekulargewichtsvergrößerten Katalysatoren werden dann in einem geeigneten Membranreaktor zur kontinuierlich betriebenen enantioselektiven Reduktion prochiraler Ketone eingesetzt.

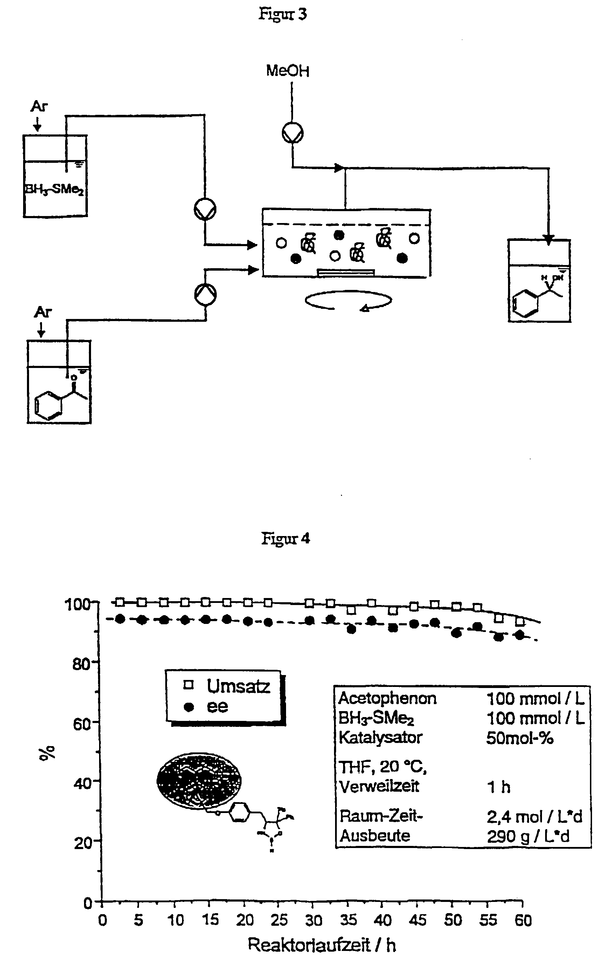

Das Fließschema eines solchen Reaktoraufbaus ist in Figur 3 wiedergegeben. Die beiden Edukte, das Boran sowie das Keton, werden - gelöst in einem geeigneten organischen Lösungsmittel, insbesondere Toluol oder Tetrahydrofuran (THF) - aus unter Schutzgas zum Ausschluß von Sauerstoff und Luftfeuchtigkeit stehenden Vorratsbehältern jeweils über eine Pumpe in den Membranreaktor gepumpt. Dieser besteht beispielsweise aus einer Rührzelle, die mit einer lösungsmittelstabilen Ultra- oder Nanofiltrationsmembran (Flachmembran) ausgerüstet ist. Es ist aber auch eine Verwendung von Hohlfasermodulen möglich. Am Reaktoraustritt wird die Reaktionslösung (über ein T-Stück) mit Methanol gequenscht, um den entstandenen chiralen Alkohol in Freiheit zu setzen und evtl. überschüssiges Boran zu vernichten.

Ein derartiger Reaktor kann über mehrere Tage, d. h. über lange Verweilzeiten, stabil betrieben werden. Ein beispielhafter Reaktorlauf ist in Figur 4 dargestellt. Es werden nach dem erfindungsgemäßen Verfahren sehr hohe Umsätze und Enantiomerenüberschüsse erreicht. Damit wird eine hohe Raum-Zeit-Ausbeute erzielt, welche ein wichtiges Kriterium für die Wirtschaftlichkeit eines Verfahrens ist.

Im Vergleich zur Verwendung freier - oder auch heterogenisierter - Katalysatoren im Satzreaktor entfällt durch die kontinuierliche Betriebsweise auch ein großer Teil der Rüstzeit. Außerdem wird die in einem vergleichbaren Satzreaktor anfallende große Menge boranhaltiger Reaktionslösung und das damit verbundene Gefahrenpotential vermieden.

Die Aufarbeitung der Produktlösung ist gegenüber einer üblichen Produktion im Satzreaktor ebenfalls deutlich vereinfacht, da der Katalysator nicht mehr abgetrennt werden muß. Die Entfernung überschüssigen Borans gelingt auf einfachem Wege: Entweder kann es nach dem Methanol-Quenching als Borsäuretrimethylester abdestilliert werden, oder es ist nach wässriger Aufarbeitung als Borsäure leicht einer alkalischen Extraktion zugänglich. Durch die hohen Umsätze - d. h. durch eine annähernd quantitative Umwandlung des Ketons in den Alkohol und Vermeiden des Auftretens von Nebenprodukten - wird auch eine Aufreinigung des Rohproduktes vereinfacht, wenn nicht sogar überflüssig.

Mit dem erfindungsgemäßen Verfahren kann eine Vielzahl von Ketonen auf wirtschaftliche Weise in die chiralen Alkohole überführt werden. Durch die Verwendung der molekulargewichtsvergrößerten, homogenlöslichen Oxazaborolidine kann die Zyklenzahl dieser Katalysatoren deutlich gesteigert werden, ohne daß - wie bei anderen Verfahren - eine Einbuße in der Enantioselektivität hingenommen werden muß. Vielmehr werden überraschenderweise zum Teil sogar bessere Enantioselektivitäten beobachtet, als mit vergleichbaren freien Katalysatoren im Satzreaktor.

Nachfolgend wird die Erfindung anhand von Beispielen näher erläutert.

### Ausführungsbeispiel:

### Darstellung von polymervergrößertem α,α-Diphenyltyrosinol:

Zu einer Lösung von 860 mmol Phenylmagnesiumbromid, hergestellt aus 22 g Magnesium und 90 ml Brombenzol, in 900 ml Tetrahydrofuran (THF) werden bei 0 °C portionsweise 21 g Tyrosinethylesterhydrochlorid (85 mmol) gegeben und über Nacht bei Raumtemperatur gerührt. Anschließend wird mit Ammoniumchloridlösung hydrolysiert. Die organische Phase wird abgetrennt und die wäßrige Phase 4x mit Ethylacetat extrahiert. Die vereinigten organischen Phasen werden über Natriumsulfat getrocknet und das Lösungsmittel abdestilliert. Zweimaliges Umkristallisieren aus Ethanol ergibt 17 g (S)-2-Amino-3-(4-hydroxyphenyl)-1,1 - diphenylpropan-1-ol (= α,α-Diphenyltyrosinol, 62 % Ausbeute). Dieses wird in Dimethylformamid mit einem Äquivalent Natriumhydrid versetzt und nach Abklingen der H₂-Entwicklung (ca. 1 Std.) mit einer äquimolaren Menge Vinylbenzylchlorid umgesetzt. Nachdem 5 Std. bei Raumtemperatur gerührt wurde, wird die Reaktionslösung auf einen Überschuß Wasser gegossen, der weiße Niederschlag abfiltriert und das Produkt aus Ethanol umkristallisiert. Man erhält (S)-2-Amino-3-(4-(vinylphenylmethoxy)-1,1- diphenylpropan-1-ol in etwa 45 % Ausbeute.

13 g dieses Monomers (29,9 mmol) werden mit 5 Äquivalenten frisch destilliertem Styrol (150 mmol) unter Verwendung von 275 mg Azobisisobutyronitril als Radikalstarter in 220 ml Toluol copolymerisiert. Die Mischung wird 50 Stunden bei 60 °C unter Argon gerührt. Dann wird das Polymer in Methanol präzipitiert. Man erhält 15,1 g Polymer, entsprechend 53 % Ausbeute. Die Molmasse beträgt laut Gelpermeationschromatographie 13 800 (Zahlenmittel).

### Durchführung einer kontinuierlichen Reduktion im Membranreaktor:

Die Reaktoranordnung für die kontinuierliche enantioselektive Reduktion entspricht dem Schema in
Figur 3. Als Vorlagegefäße dienen laborübliche Dreihalskolben, die unter Schutzgas gesetzt werden. Für die Leitungen werden Teflonschläuche verwendet. Als Pumpen kommen Pharmacia P-500 Wechselkolbenpumpen zum Einsatz. Der Membranreaktor besteht aus einer Polypropylen-Flachmembranzelle mit 10 ml Reaktionsvolumen, die von einem Magnetrührer gerührt wird. Er wird mit einer lösungsmittelstabilen Nanofiltrationsmembran MPF 50 der Firma Membrane Products ausgerüstet.

Pumpen und Reaktor werden mit absolutiertem (wasserfreiem) THF gespült. Anschließend wird über eine der Pumpen 0,5 mmol des polymergebundenen Liganden (entsprechend 50 mol-% Katalysator) - in THF gelöst - in den Membranreaktor eingespült. Nun wird der Reaktor für 1 bis 2 Stunden mit einer Lösung von Boran-Dimethylsulfid (BH₃-SMe₂) in THF gespült (200 mmol/L, 10-20 ml/Std.).
Dabei bildet sich aus dem im Membranreaktor zurückgehaltenen Aminoalkohol das Oxazaborolidin.

Dann wird über die zweite Pumpe eine Lösung von 200 mmol/L Acetophenon in THF in den Reaktor dosiert. Die Flüsse beider Pumpen werden auf 5 ml/Std. eingestellt. Damit beträgt die Verweilzeit τ = 1 Std. und die Anfangskonzentration an Keton und Boran je 100 mmol/L. Über das T-Stück am Reaktorausgang wird mit 4 ml/Std. Methanol gequencht. Der Reaktorauslauf wird in einem Fraktionssammler aufgefangen. Umsatz und ee werden gaschromatographisch bestimmt. Ein entsprechender Reaktorlauf ist in Figur 4 wiedergegeben. Es zeigte sich, daß der Reaktor über einen langen Zeitraum stabil betrieben werden kann und Umsätze bis zu 100 % erreicht werden. Zudem liefert das erfindungsgemäße Verfahren die angestrebten Enantiomerenüberschüsse von ≥ 90 % ee.

## Patentansprüche

1. Verfahren zur katalytischen, enantioselektiven Reduktion von Ketonen zu chiralen Alkoholen,
**dadurch gekennzeichnet,**
daß die Reaktion mit einem molekulargewichtsvergrößerten Katalysator in einem Membranreaktor durchgeführt wird.

2. Verfahren nach Anspruch 1,
**dadurch gekennzeichnet,**
daß als molekulargewichtsvergrößerter Katalysator ein chirales Oxazaborolidin eingesetzt wird.

3. Verfahren nach Anspruch 1 oder 2,
**dadurch gekennzeichnet,**
daß das Oxazaborolidin über den chiralen Aminoalkohol an die zur Molekulargewichtsvergrößerung verwendete Verbindung angekoppelt ist.

4. Verfahren nach Anspruch 3,
**dadurch gekennzeichnet,**
daß das Oxazaborolidin über ein Tyrosinol- oder Hydroxyprolinderivat an die zur Molekulargewichtsvergrößerung verwendete Verbindung angekoppelt ist.

5. Verfahren nach Anspruch 4,
**dadurch gekennzeichnet,**
daß als Tyrosinol- bzw. Hydroxyprolinderivat Diphenyltyrosinol bzw. Diphenylhydroxyprolinol eingesetzt wird.

6. Verfahren nach einem der vorhergehenden Ansprüche,
**dadurch gekennzeichnet,**
daß ein Polymer zur Molekulargewichtsvergrößerung eingesetzt wird.

7. Verfahren nach Anspruch 6,
**dadurch gekennzeichnet,**
daß ein Polystyrol oder Polysiloxan zur Molekulargewichtsvergrößerung eingesetzt wird.

## Claims

1. Process for the catalytic, enantioselective reduction of ketones to yield chiral alcohols,
characterised in that
the reaction is performed with an increased molecular weight catalyst in a membrane reactor.

2. Process according to claim 1,
characterised in that
a chiral oxazaborolidine is used as the increased molecular weight catalyst.

3. Process according to claim 1 or 2,
characterised in that
the oxazaborolidine is coupled to the compound used to increase molecular weight via the chiral amino alcohol.

4. Process according to claim 3,
characterised in that
the oxazaborolidine is coupled to the compound used to increase molecular weight via a tyrosinol or hydroxyproline derivative.

5. Process according to claim 4,
characterised in that
diphenyltyrosinol or diphenylhydroxyprolinol are used respectively as the tyrosinol or hydroxyproline derivative.

6. Process according to one of the preceding claims,
characterised in that
a polymer is used to increase the molecular weight.

7. Process according to claim 6,
characterised in that
a polystyrene or polysiloxane is used to increase the molecular weight.

## Revendications

1. Procédé de réduction catalytique, énantiosélective de cétones en alcools chiraux,
caractérisé en ce que
la réaction est effectuée avec un catalyseur dont le poids moléculaire a été augmenté, dans un réacteur à membrane.

2. Procédé selon la revendication 1,
caractérisé en ce que
comme catalyseur dont le poids moléculaire a été augmenté, on utilise une oxazaborolidine chirale.

3. Procédé selon la revendication 1 ou 2,
caractérisé en ce que
l'oxazaborolidine est couplée par l'aminoalcool chiral au composé utilisé pour l'augmentation du poids moléculaire.

4. Procédé selon la revendication 3,
caractérisé en ce que
l'oxazaborolidine est couplée par l'intermédiaire d'un dérivé du tyrosinol ou de l'hydroxyproline au composé utilisé pour l'augmentation du poids moléculaire.

5. Procédé selon la revendication 4,
caractérisé en ce que
comme dérivé du tyrosinol ou de l'hydroxyproline, on met en oeuvre le diphényltyrosinol ou le diphénylhydroxyprolinol.

6. Procédé selon l'une quelconque des revendications précédentes,
caractérisé en ce qu'
on utilise un polymère pour l'augmentation du poids moléculaire.

7. Procédé selon la revendication 6,
caractérisé en ce qu'
on utilise un polystyrène ou un polysiloxane pour l'augmentation du poids moléculaire.
